# EUROPEAN PATENT APPLICATION

(11) **EP 1 810 698 A1**
(43) Date of publication of application: **25.07.2007**
(21) Application number: 06250384.2
(22) Date of filing: 24.01.2006
(51) Int. Cl.: A61L 24/00

(54) **Process for producing radiopaque embolic microspheres**

(71) Applicant: Biocompatibles UK Limited, Farnham Surrey GU9 8QL (GB)
(72) Inventor: Lewis, Andrew Lennard Biocompatibles UK Limited, Farnham, Surrey GU9 8QL (GB); Goreish, Hind Hassan SidAhmed, Hounslow, Middlesex TW4 7RY (GB); Tang, Yiqing Biocompatibles UK Limited, Farnham, Surrey GU9 8QL (GB)
(74) Representative: Jones, Helen M.M.

(57) **Abstract**

A method is described for producing radiopaque polymer beads suitable for use in embolisation therapy. Dry beads, preferably dried by lyophilisation, are contacted with a solvent mixture with iodinated oil, to swell the beads. After removal of excess solvent mixture, the beads are dried to remove solvent and then suspended in aqueous storage liquid and sterilised by heating.

## Description

The present invention relates to the preparation of storage stable microspheres useful in embolisation therapy which are visible under X-ray.

Embolisation therapy involves the introduction of an agent into the vasculature in order to bring about the deliberate blockage of a particular vessel. This type of therapy is particularly useful for blocking abnormal connections between arteries and veins (such as arteriovenous malformations, or AVMs), and also for occluding vessels that feed certain hypervascularised tumours, in order to starve the abnormal tissue and bring about its necrosis and shrinkage.

Lipiodol is iodinated oily X-ray contrast medium composed of about 40% iodinated poppy-seed oil. It can be used in certain radiological investigations where it is desirable to outline a viscous or other structure with directly instilled radio-opaque material. It is also suitable for introduction into narrow channels and may therefore be used in embolisation procedures. Lipiodol has a long history and wealth of toxicity data available in literature with no evidence of adverse effects and few complications.

Lipiodol oil is well-established for use in the medical industry and is commonly used in diagnostic hysterosalpinography (HSG). HSG is radiography (or x-ray) of the uterus and fallopian tubes used for the detection of gynaecological problems, particularly those affecting female fertility. The procedure was first carried out in 1910 and there are claims that pregnancy rates increase after the procedure has been carried out.

Lipiodol is also used for lymphography, which is the examination of the lymph nodes and lymphatic vessels, in particular for the prognostic diagnosis of leukaemia and Hodgkin's and non-Hodgkin's lymphoma.

Lipiodol is also used for sialography, for detecting disorders of the salivary glands or ducts.

Another oil having similar properties which is an iodinated (37% wt) fatty acid ethyl esters is Ethiodol. This contains a mixture of the mono-iodo- and di-iodo derivatives, mainly stearates. This is again derived from poppy seed oil and contains (non-iodinated) poppy seed oil to stabilise the mixture. It has a specific gravity of 1.28 g/ml at 15°C.

Attempts have been made to render embolic particles radiopaque. For instance Thanoo et al, in J. App. Biomat. 2, 67-72 (1991) describe the encapsulation of barium sulphate in hydrogel materials useful for embolisation therapy. Microspheres based on crosslinked polyvinyl alcohol containing 40 or 60% barium sulphate could be produced. It was asserted that the particles were clearly visible in x-ray images. Barium sulphate was firmly trapped inside the microspheres. The incorporation of barium sulphate could be carried out without adverse effect on the microsphere size.

Thanoo also describe incorporation of another radiopaque material, methyl iothalamate. However incorporation of this material was found to give rise to larger droplets in the PVA crosslinking reaction and hence larger microspheres. The methyl iothalamate was, however, firmly entrapped in the microspheres and did not migrate upon sonication or steam sterilisation.

Others have attempted to incorporate radiopaque monomers, that is iodinated monomers, into mixtures of ethylenically unsaturated monomers used to form hydrogel type microsphere embolic agents. However it has been difficult to incorporate sufficient monomer to achieve adequate radiopacity. Furthermore ensuring that the products of such polymerisation reactions are free of unreacted potentially toxic monomers is difficult.

In current clinical use, none of the routinely used embolic microsphere products contain preloaded radiopaque materials. Instead the microsphere products are pre-mixed with contrast medium, such as lipiodol, immediately prior to administration. However the mixtures tend to be unstable, at least in part because of the different densities of the beads, water and lipiodol, and the immiscibility of lipiodol with water. Contacting lipiodol with water-swollen beads does not result in entrapment of the oil in the beads so that the beads cannot themselves be visualised after administration, but rather only the liquid bolus which is administered is visible for a short period. Lipiodol is therefore used off-label as an embolisation agent in itself, whereby its viscous nature tends to trap in the capillary bed and thus slows down the blood flow. This is then normally followed by conventional embolisation with particles or microspheres. The oil partitions into tumourous tissue and allows long term visualisation by CT.

It is generally desirable for embolic beads to have a relatively high equilibrium water content in order that they are sufficiently compressible to be delivered down a microcatheter. This means that the beads must either have macropores and/or must be formed of very hydrophilic polymers. Since lipiodol is an oil, it is not particularly compatible with these hydrophilic polymer materials. Accordingly when aqueous suspensions of swollen beads are mixed with lipiodol there is little or no penetration of the iodinated oil into the matrix.

Tamura, S., et al., "Lipiodolized Gelatin Sponge: A Simple Method to Make Gelatin Sponge Radiopaque", Diagnostic Radiol. (1998), 16(1), 13-15, describe tests to soak gelatin sponge particles in lipiodol. The particles were obtained by chopping a block of sponge into 1 to 2 mm particles and contacted with lipiodol then used for renal embolizations. The radiopacity of the particles *in vitro* in water and *in vivo* was monitored after 24 hours. Although it was concluded that the sponge particles were rendered radiopaque *in vitro* and *in vivo* it is not clear that lipiodol is in fact absorbed into the particles of sponge. Indeed the author suggests that the injected material included lipiodol droplets and such droplets could cause complications if administered to certain arteries.

According to the invention there is provided a new process for forming radiopaque embolic beads including the steps:
a) contacting substantially dry water-swellable beads, which are capable of swelling in phosphate buffered saline at 20°C to an equilibrium water content of at least 40%, with a loading liquid comprising a pharmaceutically acceptable iodinated oil for a period of at least 5s to produce oil-loaded beads;
b) recovering the oil-loaded beads from any excess extra-particulate liquid mixture;
c) contacting the beads after step b) with an aqueous storage liquid in excess of the amount required to swell the beads to equilibrium; and
d) sterilising the beads in the storage liquid by heating to a temperature of at least 90°C for at least 5 seconds.

The beads have a diameter in the range 25 to 1500 µm, preferably in the range 50 to 1200 µm, for instance in the range 100 to 1200 µm.

In the invention, the term bead is intended to cover particles of all shapes, for instance rod shapes, cubes, irregular and non-uniform shapes. However the invention is of most benefit where the beads are spherical, spheroidal or pellet shaped, or disk shaped. In non-spherical particles, such as pellets, spheroids or disks, the maximum dimension is preferably no more than three times the minimum diameter, and preferably less than two times the minimum diameter, for instance around 1.5 or less. The size limitations mentioned above are determined by testing a sample of the swellable beads under conditions in which the beads are swollen to equilibrium in phosphate buffered saline at room temperature and the sizes are measured using an optical microscope.

The compositions are preferably provided with a particle size specification which defines the spread of diameters. Preferably the beads are graded into calibrated size ranges for accurate embolisation of vessels. The particles preferably have sizes when equilibrated in water at 37°C, in the range 100 to 1500µm, more preferably in the range 100 to 1200µm. The calibrated ranges may comprise beads having diameters with a bandwidth of about 100 to 300µm. The size ranges may be for instance 100 to 300µm, 300 to 500µm, 500 to 700µm, 700 to 900µm and 900 to 1200µm.

Preferably the polymer comprises alcoholic hydroxyl groups or acylated derivatives thereof. In one embodiment polymers are used which are derived from natural sources, such as albumin, alginate, gelatin, starch, chitosan or collagen, all of which have been used as embolic agents. In a preferred embodiment the polymer is substantially free of naturally occurring polymer or derivatives. It is preferably formed by polymerising ethylenically unsaturated monomers including monomers having hydroxyalkyl or acyloxyalkyl groups in the presence of di- or higher-functional crosslinking monomers. The ethylenically unsaturated monomers may include an ionic (including zwitterionic) monomer. The hydroxyl group or acylated derivative is believed to undergo trans- or inter-esterification with the ester moiety of the oil whereby the iodinated fatty acyl group is covalently linked to the polymer although the applicant is not to be bound by this theory.

Copolymers of hydroxyethyl methacrylate, acrylic acid and cross-linking monomer, such as ethylene glycol dimethacrylate or methylene bisacrylamide, as used for etafilcon A based contact lenses may be used. Copolymers of N-acryloyl-2-amino-2-hydroxymethyl-propane-1,3-diol and N,N-bisacrylamide may also be used.

Other polymers are cross-linking styrenic polymers e.g. with ionic substituents, of the type used as separation media or as ion exchange media.

Another type of polymer which may be used to form the water-swellable water-insoluble matrix is polyvinyl alcohol crosslinked using aldehyde-type crosslinking agents such as glutaraldehyde. For such products, the polyvinyl alcohol (PVA) may be rendered ionic by providing pendant ionic groups by reacting a functional ionic group containing compound with the hydroxyl groups. Examples of suitable functional groups for reaction with the hydroxyl groups are acylating agents, such as carboxylic acids or derivatives thereof, or other acidic groups which may form esters.

The invention is of particular value where the polymer matrix is formed from a polyvinyl alcohol macromer, having more than one ethylenically unsaturated pendant group per molecule, by radical polymerisation of the ethylenic groups. Preferably the PVA macromer is copolymerised with ethylenically unsaturated monomers for instance including a nonionic and/or ionic monomer including anionic monomer.

The PVA macromer may be formed, for instance, by providing PVA polymer, of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Other methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, an (alk)acrylaminoalkyl moiety. Example 1 describes the synthesis of an example of such a macromer known by the approved named nelfilcon B. Preferably the PVA macromers have about 2 to 20 pendant ethylenic groups per molecule, for instance 5 to 10.

Where PVA macromers are copolymerised with ethylenically unsaturated monomers including an ionic monomer, the ionic monomer preferably has the general formula I

Y¹BQ¹ I

in which Y¹ is selected from 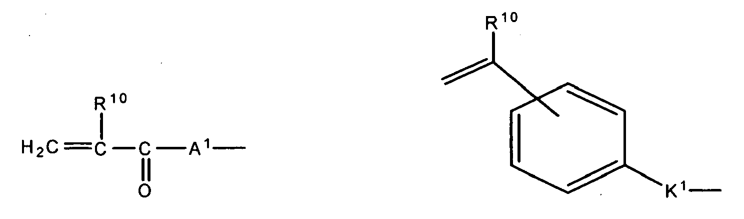 CH₂=C(R¹⁰)-CH₂-O-, CH₂=C(R¹⁰)-CH₂ OC(O)-, CH₂=C(R¹⁰)OC(O)-, CH₂=C(R¹⁰)-O-, CH₂=C(R¹⁰)CH₂OC(O)N(R¹¹)-, R¹²OOCCR¹⁰=CR¹⁰C(O)-O-, R¹⁰CH=CHC(O)O-, R¹⁰CH=C(COOR¹²)CH₂-C(O)-O-, 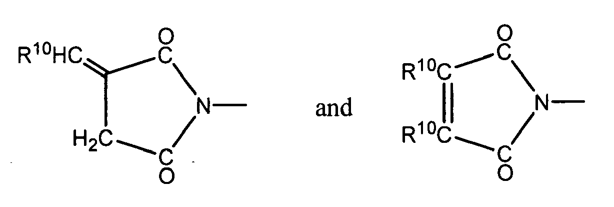 wherein:
R¹⁰ is hydrogen or a C₁-C₄ alkyl group;
R¹¹ is hydrogen or a C₁-C₄ alkyl group;
R¹² is hydrogen or a C₁₋₄ alkyl group or BQ¹ where B and Q¹ are as defined below;
A¹ is -O- or -NR¹¹ -;
K' is a group -(CH₂)ᵣOC(O)-, -(CH₂)ᵣC(O)O-, -(CH₂)ᵣOC(O)O-, -(CH₂)ᵣNR¹³-, -(CH₂)ᵣNR¹³C(O)-, -(CH₂)ᵣC(O)NR¹³-, -(CH₂)ᵣNR¹³C(O)O-, -(CH₂)ᵣOC(O)NR¹³-, -(CH₂)ᵣNR¹³C(O)NR¹³- (in which the groups R¹³ are the same or different), -(CH₂)ᵣO-, -(CH₂)ᵣSO₃-, or, optionally in combination with B, a valence bond and r is from 1 to 12 and R¹³ is hydrogen or a C₁-C₄ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if Q¹ or Y¹ contains a terminal carbon atom bonded to B a valence bond; and
Q¹ is an ionic group.

Such a compound including an anionic group Q¹ is preferably included.

An anionic group Q¹ may be, for instance, a carboxylate, carbonate, sulphonate, sulphate, nitrate, phosphonate or phosphate group. The monomer may be polymerised as the free acid or in salt form. Preferably the pKₐ of the conjugate acid is less than 5.

A suitable cationic group Q¹ is preferably a group N⁺R¹⁴₃, P⁺R¹⁵₃ or S⁺R¹⁵₂ in which the groups R¹⁴ are the same or different and are each hydrogen, C₁₋₄-alkyl or aryl (preferably phenyl) or two of the groups R¹⁴ together with the heteroatom to which they are attached from a saturated or unsaturated heterocyclic ring containing from 5 to 7 atoms the groups R¹⁵ are each OR¹⁴ or R¹⁴. Preferably the cationic group is permanently cationic, that is each R¹⁴ is other than hydrogen. Preferably a cationic group Q is N⁺R¹⁴₃ in which each R¹⁴ is C₁₋₄-alkyl, preferably methyl.

A zwitterionic group Q¹ may have an overall charge, for instance by having a divalent centre of anionic charge and monovalent centre of cationic charge or vice-versa or by having two centres of cationic charge and one centre of anionic charge or vice-versa. Preferably, however, the zwitterion has no overall charge and most preferably has a centre of monovalent cationic charge and a centre of monovalent anionic charge.

Examples of zwitterionic groups which may be used as Q in the present invention are disclosed in WO-A-0029481.

Where the ethylenically unsaturated monomer includes zwitterionic monomer, for instance, this may increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-methacryloyloxy-2'-trimethylammonium ethyl phosphate inner salt (MPC).

In the monomer of general formula I preferably Y¹ is a group CH²=CR¹⁰COA-in which R¹⁰ is H or methyl, preferably methyl, and in which A¹ is preferably NH. B is preferably an alkanediyl group of 1 to 12, preferably 2 to 6 carbon atoms. Such monomers are acrylic monomers.

There may be included in the ethylenically unsaturated monomer diluent monomer, for instance non-ionic monomer. Such a monomer may be useful to control the pKₐ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and di-hydroxy-substituted alkyl(alk) acrylates and -(alk) acrylamides, vinyl lactams, styrene and other aromatic monomers.

In the polymer matrix, the level of anion is preferably in the range 0.1 to 10 meq g⁻¹, preferably at least 1.0 meq g⁻¹. Preferred anions are derived from strong acids, such as sulphates, sulphonates, phosphates and phosphonates.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to 100 mole%, preferably at least 25 mole%.

The crosslinked polymer may be formed as such in particulate form, for instance by polymerising in droplets of monomer in a dispersed phase in a continuous immiscible carrier. Examples of suitable water-in-oil polymerisations to produce particles having the desired size, when swollen, are known. For instance US 4,224,427 describes processes for forming uniform spherical beads (microspheres) of up to 5 mm in diameter, by dispersing water-soluble monomers into a continuous solvent phase, in a presence of suspending agents. Stabilisers and surfactants may be present to provide control over the size of the dispersed phase particles. After polymerisation, the crosslinked microspheres are recovered by known means, and washed and optionally sterilised. Preferably the particles eg microspheres, are swollen in an aqueous liquid, and classified according to their size.

In the invention the loading liquid may consist substantially only of oil. Preferably it additionally contains an organic solvent which is miscible with or emulsifiable with the iodinated oil. It is preferred that solvent is capable of swelling the beads and that it is volatile relative to the oil.

The organic solvent is preferably selected from monohydric C-1-12 aliphatic alcohols and is preferably selected from ethanol and propanol. The solvent should preferably be volatile relative to the oil. For instance it should preferably have a boiling point of less than 90°C, more preferably less than 80°C, in order to allow removal under mild conditions, e.g., involving evaporation at low temperatures and preferably low pressures.

Iodinated oil comprises at least 30% by weight iodine, preferably in the range 35 to 45% iodine. Such levels of iodine tend to provide an oil with a relatively high density, for instance more than 1 g/ml. Preferred oils are based on poppy seed oil, and are for instance lipiodol or ethiodol. Generally the iodinated poppy seed oils contain predominantly mono- and di-iodo substituted stearate esters. The esterifying moiety is generally a lower alkyl group, for instance ethyl. The nature of the esterifying group may be controlled by subjecting naturally occurring oils to transesterification techniques or interesterification techniques.

The process of the present invention may be carried out on beads, starting from a commercially available form of the beads, for instance an aqueous suspension. Where the beads are provided in such a form, they must be subjected to an initial step of drying, in order to provide the starting material for step a). An aqueous suspension of beads may be dried, for instance, by a process involving normal solid/liquid separation steps such as filtration and/or centrifugation, followed by a step of further removing water e.g. by evaporation or sublimation. One way of removing water involves spray drying of a slurry, another way involves fluid bed drying and another way involves the use of dehydrating solvents, such as acetone which deswell the polymer, followed by solvent evaporation. Most conveniently the drying step involves lyophilisation which leaves the dried beads with a macroporous structure which has desirable properties for absorbing the iodinated oil. It is preferred therefore that the process of the invention involves a step of lyophilising water-swollen beads to form the substantially dry beads used as the starting material for step a).

In the process of the invention it is an essential step that the final product is sterilised by a heating process in the presence of water. This renders the end product suitable for administration, for instance in an embolisation procedure. Sterilisation is carried out by heating to a suitably raised temperature, of at least 90°, preferably at least 100°C, and more preferably to a higher temperature than 100°C under pressure. The heating period should be selected to ensure that adequate sterilisation is achieved. Particularly suitable sterilisation conditions involve autoclaving, that is under raised pressure, at a temperature of around 120°C for at least 5 minutes, preferably around 15 minutes or more. Under these conditions it has been found that the radiopaque oil is not adversely affected. Without wishing to be bound by theory it is believed that, under these conditions a trans- or inter-esterification takes place whereby the hydroxyl or acyloxy groups are fatty acylated with iodinated acyl groups. This binds the radiopaque iodine moieties to the polymer so that the beads remain radiopaque during and after delivery.

According to a preferred aspect of the invention there is provided a process including a step involving trans- or inter-esterification between the hydroxyl group on the polymer and the ester group of the iodinated oil whereby iodinated acyl groups become bound to the polymer through ester linkages.

The level of iodinated oil in the loading liquid is up to and includes 100%. Where the liquid is a mixture the ratio of solvent to iodinated oil is in the range 99:1 to 1:99, preferably in the range 95:5 to 5:95, more preferably in the range 9:1 to 1:9, for instance in the range 5:1 to 1:5.

The iodinated oil is generally used in an amount relative to the beads such that the product of step b) contains at least 10% by weight iodinated oil, preferably at least 20% by weight iodinated oil, and more preferably at least 30% iodinated oil. The loading level is controlled by controlling the amount of loading liquid compared to dry beads used in step a), as well as the relative amounts of solvent and iodinated oil in the loading liquid mixture where used. In general the amount of loading liquid used in step a) is in excess of that which will fully penetrate the beads under the loading conditions. This means that, even after an extended contact period there will be a continuous liquid mixture on the exterior of the beads.

In step b), the swollen beads are recovered from this excess liquid mixture. Such separation is by usual solid/liquid separation techniques. Where the densities are appropriate, this may involve centrifugation. Preferably it involves filtration. After such solid/liquid separation steps, some and preferably at least 95% of any remaining organic solvent is removed from the beads, since it is generally preferred that the embolic mixture administered to a patient does not contain such solvent. However for some solvents it may be possible to leave some or all of the solvent in the products. Removal of organic solvent is generally achieved by evaporation, for instance a raised temperature or reduced pressure for instance in a fluid bed drier. In order that this drying step does not remove oil, the conditions should be such that low or no detectable levels of the iodinated oil are evaporated.

We have found that the use of a solvent in conjunction with an iodinated oil in the swelling step, step a), satisfactory loading levels are achieved whereby the iodinated oil is absorbed into the beads in such a form that, during the subsequent steps, it does not migrate to the surface, but remains distributed throughout the beads. This may be due to transesterification between the oil ester and the hydroxyl groups on the polymer. The oil is stable during the subsequent drying and sterilisation steps. The final beads, when administered into the circulation system, are visible using x-ray, at safe, low radiation levels. The oil-loaded beads also have very useful suspension properties in that the iodinated oil, being of higher density than water, when absorbed in the beads, prevents them floating in the aqueous storage liquid.

We have further found that it is possible to incorporate drugs into the embolic beads. There are several ways of incorporating drug. For instance drug may be incorporated prior to formation of the dry beads for producing the starting material for step a) of the process. In another embodiment, drug may be loaded with the aqueous storage liquid in step c). In a preferred embodiment, however, drug is dissolved into the loading liquid of the iodinated oil and optional organic solvent, and is absorbed into the water-swellable beads along with the iodinated oil. This process is suitable for use with active ingredients which are soluble in the iodinated oil directly or, more likely, which are soluble in the organic solvent. Examples of drug for which the process has been carried out successfully include ibuprofen, paclitaxel and dexamethasone.

The figures relate to the examples which follow:

Briefly, the figures illustrate the following:
Figure 1 is a photograph of the beads produced in Example 1;
Figures 2 and 3 show the beads of example 2 under fluoroscopy in a catheter tube, using 100% and 30% lipiodol, respectively;
Figure 4 is a micrograph of the results of Example 4;
Figure 5 shows FT-IR spectra of Bead Block, Lipiodol and the BB-lipiodol product of example 1, as described in Example 5;
Figures 6A and B show the results of Example 6;
Figure 7 is a micrograph of the Bead Block beads used in the Examples in swollen form before being dried and subjected to the Lipiodol loading;
Figures 8A and E are histograms of the size distribution after loading with Lipiodol and IBU as described in Example 7;
Figures 9 A and B show IBU elution profiles for beads produced as in Example 7;
Figure 10 shows the results for compression tests of Lipiodol - IBU loaded beads as described in Example 9;
Figure 11 shows the IBU elution profile for IBU from Contour SE beads loaded with lipiodol and IBU as described in Example 10;
Figure 12A-C show micrographs of three commercial embolic products loaded with lipiodol and IBU according to the present invention (Example 10);
Figure 13 is an elution profile for PTX from two of the products of the Lipiodol/PTX loading method of Example 11;
Figures 14A and B shows micrographs of Bead Block loaded with dexamethasone in the absence and presence (respectively) of Lipiodol.
Figures 15A and 15B show a profile of IBU uptake from IBU-Lipiodol for an anion and cation exchange resin (respectively), as described in Example 14.

The invention is illustrated in the following examples:

### Example 1: Preparation of Lipiodol loaded Microspheres (L/Ethanol Bead)

Lipiodol loaded beads were prepared using Bead Block beads. The production of such beads is described in WO2004/000548 Example 1 as the "low AMPS" product. Briefly an aqueous mixture of polyvinyl alcohol macromer having acetal-linked ethylenically unsaturated groups and 2-acrylamido-2-methyl-propane sulphonate in a weight ratio of about 12:1 is suspended in a continuous phase of butyl acetate containing cellulose acetate butyrate stabiliser with agitator and is radically polymerised using redox initiation to form beads, which are washed, dyed and sieved into size fractions including the 500-700µm fraction and 700-900µm fraction used in subsequent examples.

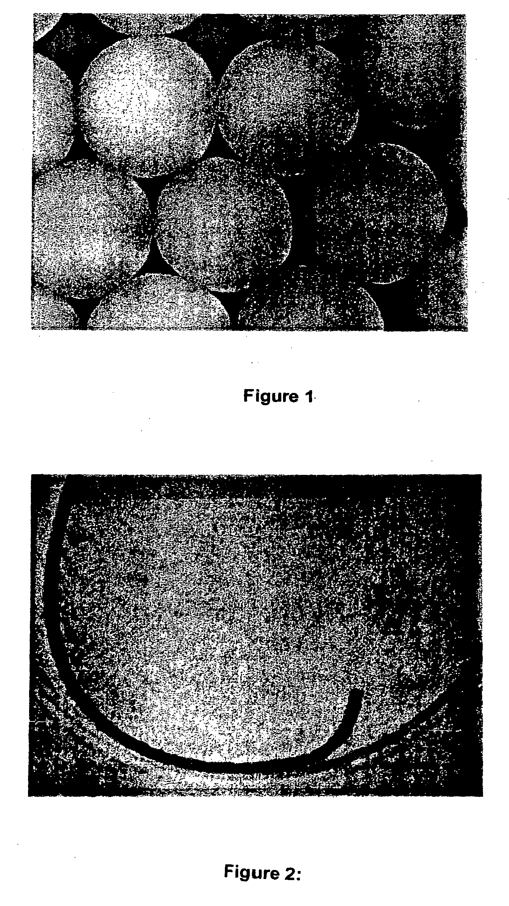

Bead Block is first partitioned in 2mls into glass vials. The excess saline is then removed and then the beads were lyophilised. 33% Lipiodol/ethanol solution is prepared. 1ml of the solution is then added to the lyophilised beads and left to load for 1 h (static). The excess loading solution is then removed and the bead sample dried overnight at 45°C in a vacuum oven. To the dried samples, 2ml water was added to hydrate the beads and they were then sterilized by autoclaving at 121°C for 15 minutes. Beads were blue/white to the naked eye. When viewed under microscope they appeared as opaque spherical beads (Fig. 1).

### Example 2: Preparation of Lipiodol loaded Microspheres (Pure L Bead)

Lipiodol loaded beads were prepared using Bead Block beads. Bead Block is first partitioned in 2mls into glass vials. The excess saline is then removed and then the beads were lyophilised. 1ml of pure Lipiodol is then added to the lyophilised beads and left to load for 1 hour (static). The excess lipiodol is then removed and the bead sample dried overnight at 45°C in a vacuum oven. To the dried samples, 2ml water was added to hydrate the beads and they were then sterilized by autoclaving at 121°C for 15 minutes.

The product had oily droplets in vial and the beads were white/blue in colour.

### Example 3: Loading with different Lipiodol (L) concentrations

Different concentrations of L in lipiodol/ethanol mixture were used for loading (1%, 5%, 10%,30%, 50%,70%, 90% and 100%). Samples were prepared as in example 1 using varying the concentration of L with ethanol. Beads were viewed using colour view camcorder III. Images. There was no distinction in the appearance of the beads except for 1 % where the beads were not all homogeneous in colour. No distinction in the appearance of the beads at all the other concentrations of L. Beads with lower concentrations of L however, had excess water in the final product indicating that L increases the uptake of water by the beads.

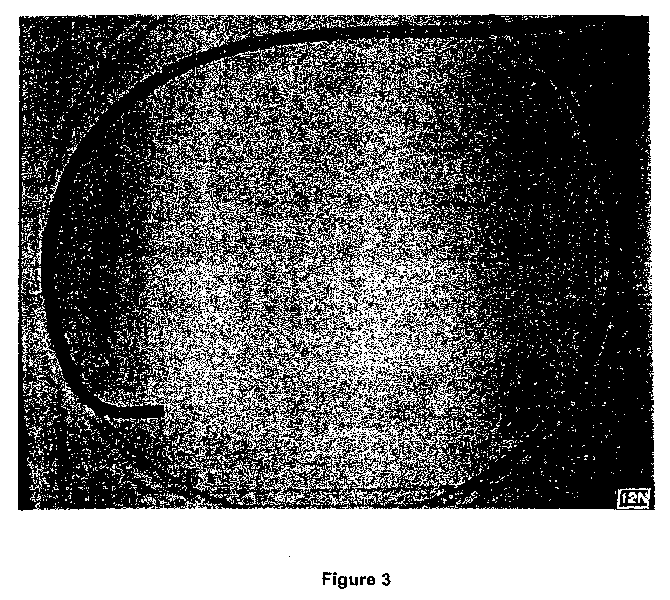

Beads with concentrations of 5% and greater were viewed under X-ray (fluoroscopy). All concentrations were visible under X-ray. No difference observed between the different concentrations. Beads appeared as aggregates of opaque beads (Fig. 2 and 3).

### Example 4: Drying by acetone and loading with 10%Lipiodol (L)

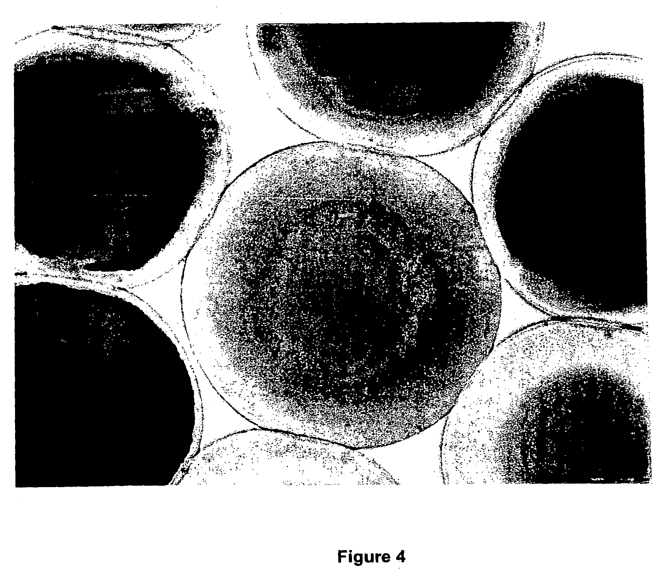

Bead Block beads were washed in acetone for 30mins then a final wash and left overnight. Excess acetone was removed and samples were dried for 1 hr. Dried beads were loaded with 10% lipiodol/ethanol for 1 hr. Excess loading solution removed and samples dried for 1 hr at 50°C in vacuum oven. Loaded beads were hydrated with water and autoclaved 121°C for 15 minutes. Beads were not loaded with Lipiodol homogeneously with some beads blue/white in colour others were blue i.e. unloaded. This was confirmed when viewed under microscope (Fig. 4). This proves that microporus structure created by lyophilisation is important for the loading of lipiodol into the beads which can be achieved by freeze drying.

### Example 5: FT-IR study of Bead Block with and without Lipiodol

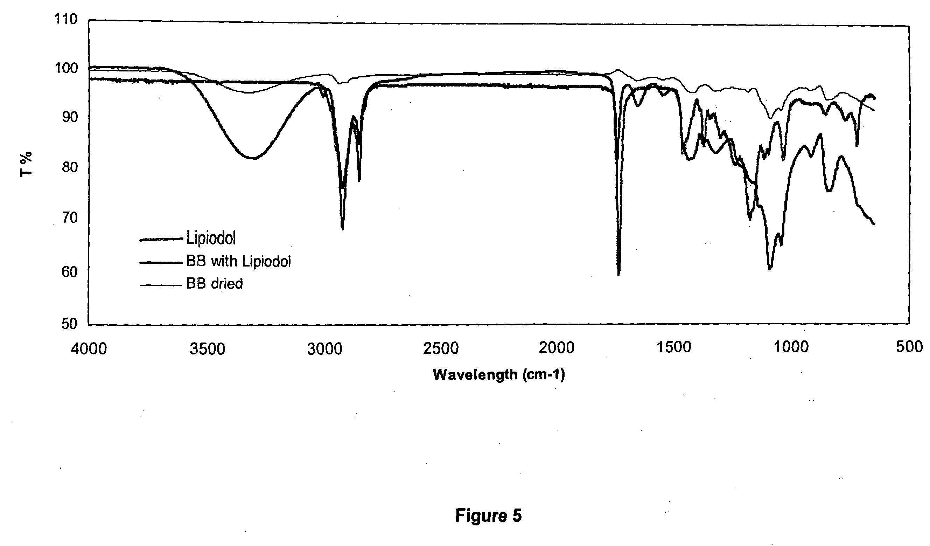

Bead Block beads loaded with Lipiodol were prepared using the method described in Example 1. The results are shown in Fig. 5. These results show a change in the spectrum after sterilisation consistent with a transesterification taking place between the alcohol derived moiety of the ester oil and the hydroxyl groups of the PVA. Autoclaving is not essential for this reaction to occur but it does accelerate the process. The transesterification is illustrated as seen in Figure 5 by a 10 cm⁻¹ shift in the position of the carbonyl stretching band.

### Example 6: Preparation of IBU-Lipiodol loaded microspheres (IBU-LBB)

The beads were prepared using the same method as in example 1 with two exceptions. The loading solution was composed of 33% Lipiodol (L) in lbuprofen/ethanol solution with Ibuprofen (IBU) at a concentration of 125 mg/ml to give a final loading of 25 mg/ml bead. The bead samples were shaken on a plate shaker set to 150rpm for 1 hour. Excess loading solution was removed and samples were dried overnight in vacuum oven set to 50°C. Loaded beads were then hydrated in 2ml water and steam sterilised as in example 1.

The first microspheres produced using this method (IBU-LBU) were in slurry form, and were blue/white beads to the naked eye. They did not stick to the glass vials and no white crystal formation on the bead surface was apparent.

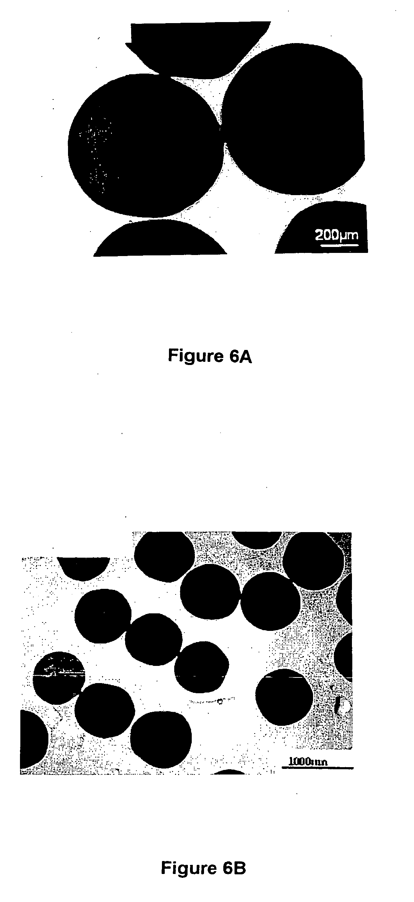

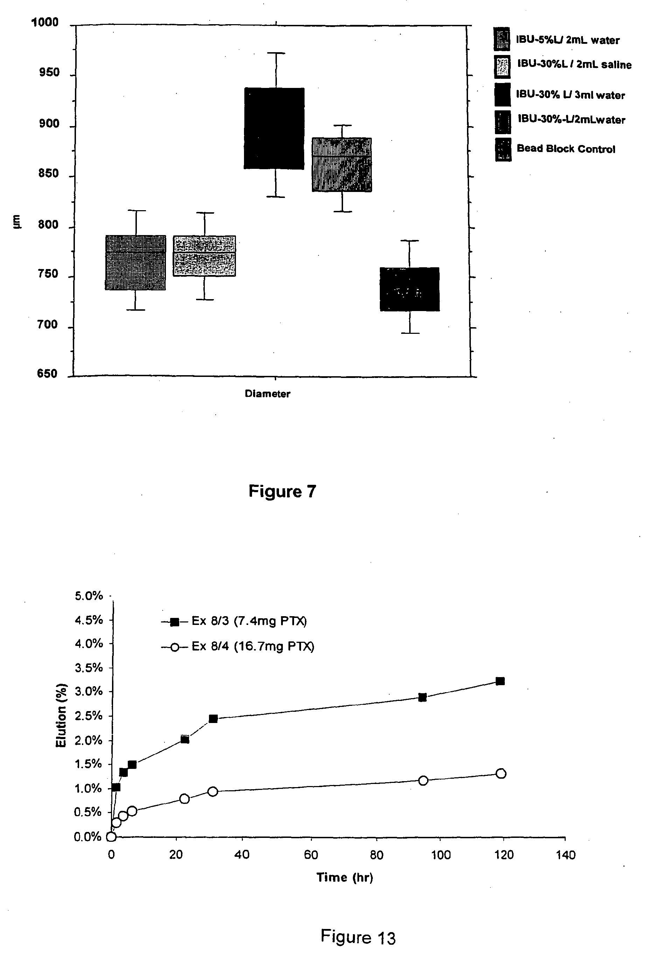

Size and Image analysis: The beads were sized using an optical microscope and visualised using a colour view III camcorder. The beads recovered their spherical shape prior to lyophilisation, and were seen to be opaque (white/blue to the naked eye) with a homogenous appearance (Fig. 6A and 6B). The size distribution of these microspheres was comparable to the starting beads (Fig. 7).

Elution: The hydration water was removed and placed in a HPLC vial to determine the drug loss during manufacturing (neat). Each vial (2ml of beads) was eluted into 200 ml of PBS for 24 hours. At predetermined time points (10, 20, 40, 60 minutes and 2, 4 and 24 hours), 1ml of beads was removed and placed into HPLC vials to determine drug eluted, degradation and purity. The amount of drug loaded into the beads was similar to that without adding Lipiodol. IBU purity was >99% showing that there was no interference with IBU. Image of the beads after elution showed opaque beads and they were not different from that of the IBU loaded beads (Fig.6B). This suggested that lipiodol uptake by the beads contributes to the opaque colour and it does not elute in PBS.

### Example 7: Effect of hydration media, type and volume using beads loaded with IBU and 5% and 30% Lipiodol concentrations

This example was conducted to examine the degree of water uptake by beads and the effect of the hydration media used. Bead Block of size 700-900 µm was loaded using IBU ethanol solutions containing either 5% or 30% Lipiodol for 1 hour using the same general technique as Example 6. The excess loading solution was removed and samples were dried overnight in vacuum oven set at 50°C. Samples were hydrated using either 2ml water, 3ml water or 2ml saline and then steam sterilized at 121°C for 15 minutes.

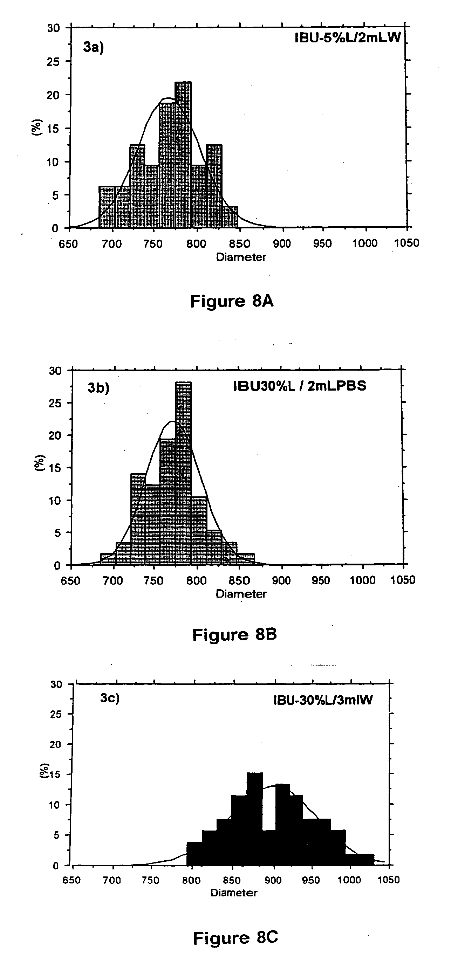

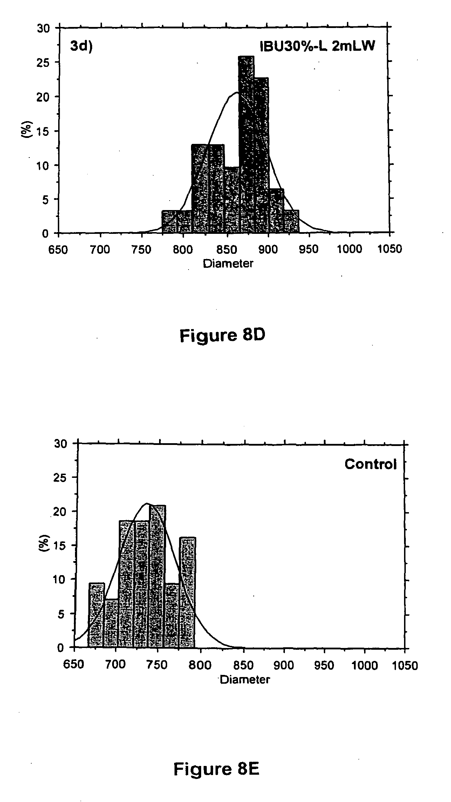

Size was measured as described in Example 6. Histograms of the size distributions are shown in figures 8A-8E. There was a statistically significant difference (p<0.0001) between all groups compared to the control beads (prior to lyophilisation and loading). No significant difference was found between using 5% lipiodol 2ml water and 30% lipiodol in 2ml saline (p=0.5158). In both instances, the size ranges were within the acceptance criteria of the size distribution with an overall shift towards the higher beads size (Fig. 7).

### Example 8: Elution on IBU-5%LBB and IBU-30%LBB on beads 500-700 µm and 700-900 µm

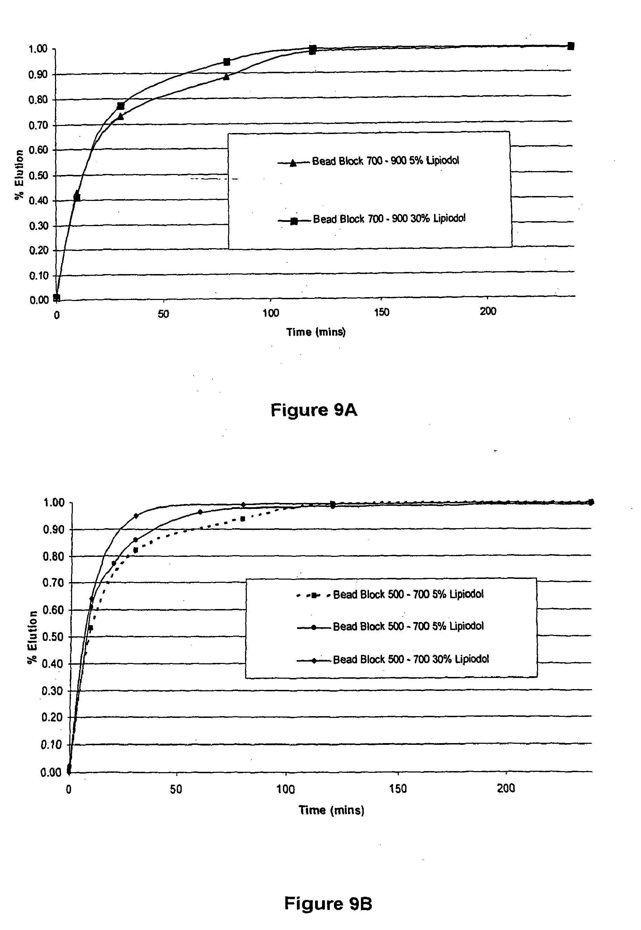

As per Example 7, the hydration water was removed and placed in a HPLC vial to determine the drug loss during manufacturing (neat). Each vial (2ml of beads) was eluted into 200 ml of PBS for 24 hours. At predetermined time points (10, 20, 40, 60 minutes and 2, 4 and 24 hours), 1ml of beads was removed and placed into HPLC vials to determine drug eluted, degradation and purity. Results (Figure 9) demonstrated that for the size 700-900 µm beads there was no difference in elution rate between the beads with 5% Lipiodol or those with 30%. Figure 9a shows that for both Lipiodol concentrations, >99% of the total IBU dose is eluted in the first 2 hours for beads size 700-900 µm. Similarly for the 500-700 µm range >99% is eluted in the first 2 hours however, there was a small shift to a faster elution with the 30% Lipiodol (Fig.9B).

### Example 9: Loading with IBU and different concentrations of Lipiodol concentrations

Bead Block of size 700-900 µm was loaded IBU ethanol solutions coating: 5%, 30%, 50%, 80%, 90% Lipiodol for 1 hour. The excess loading solution was removed and samples were dried overnight in vacuum oven set at 50°C. Samples were hydrated using 2 ml water and then steam sterilized at 121°C for 15 minutes.

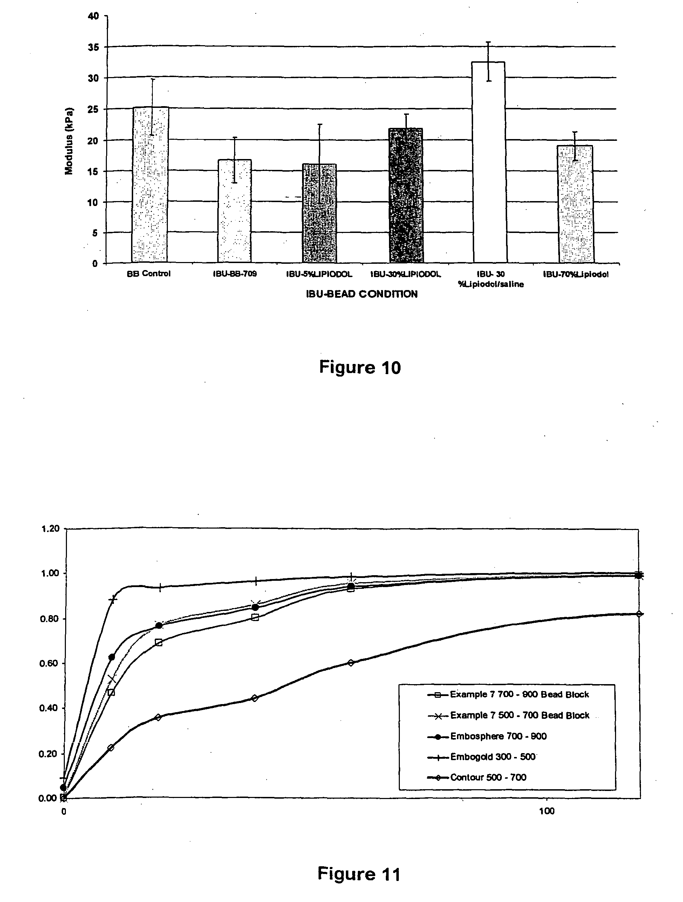

Compression testing of IBU-beads with 5%, 30% and 70% Lipiodol is shown in figure 10, and demonstrates that IBU-LBB beads at all concentrations of Lipiodol are similar to the comparative product without Lipiodol and were all more compressible than Bead Block when it is hydrated in water. When saline was used for hydration of 30% Lipiodol IBU-LBB, the compression was higher than unloaded control.

### Example 10: Loading other commercially available embolic beads

Commercially available embolic beads including: Embosphere (a trisacryl/gelatin microsphere coated with collagen), Embogold, Contour SE (a non-ionic cross-linked polyvinyl alcohol) microsphere were loaded with IBU using one loading solution of IBU/ethanol/5% Lipiodol (125mg/ml). The loading procedure is as described in Example 7.

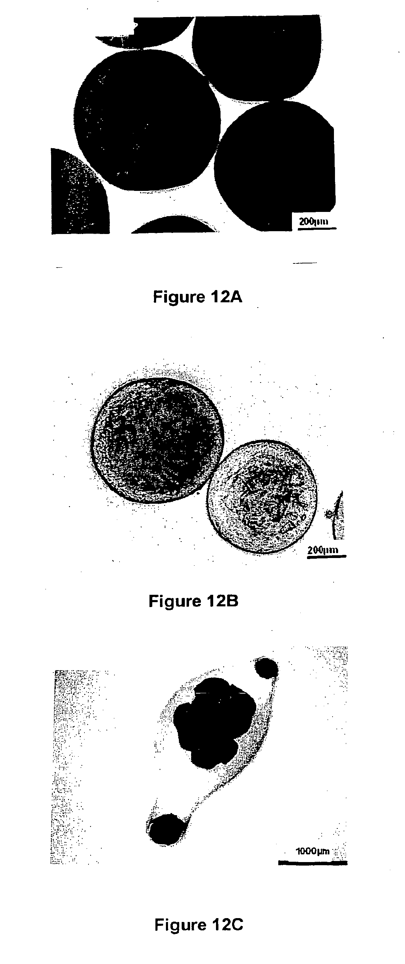

Visually beads did not change colour after loading except for Bead Block, which changed to the typical blue/white colour. Embosphere showed high tendency to stick to the glass vial whereas Contour SE showed a great deal of aggregation. Both stickiness and aggregation are associated with crystals of IBU on the surface of the beads. Images after elution have shown that Embosphere was slightly opaque (Fig. 12B) and Embogold was clear red (compared to very opaque Bead Block (Fig. 12A)) indicating lower uptake of Lipiodol and hence reduced inhibition for crystal formation. Contour SE was opaque but most beads were in clumps enclosed in oil capsule (Fig. 12C). This suggests that the bead opaque colour was due to IBU uptake. The aggregation may have been caused by IBU on the bead surface following the lyophilisation stage. The enclosure of Contour SE by Lipiodol was demonstrated by the slow elution of IBU (Fig. 11), whereas all other bead types eluted >98% IBU in first 2hours.

### Example 11: Paclitaxel (PTX) loading into Lipiodol Bead Block

PTX-loaded beads were prepared using the same method described in Example 2. Bead Block (500-700 µm and 700-900 µm) was first partitioned in 2 ml into glass vials. The excess saline was then removed, and the beads were lyophilised. PTX-Lipiodol-ethanol solution was prepared to the required final concentration, and subsequently added to the lyophilised beads and left to load for 1 hour on a plate shaker with 150 rpm. The excess loading solution was then removed and the bead sample dried overnight at 45°C in a vacuum oven. To the dried samples, 2ml water was added to hydrate the beads and they were then steam-sterilized by autoclaving at 121°C for 15 minutes.

The PTX loading content was determined by using DMSO to extract loaded PTX under ultrasonication. The extracted solution was examined by HPLC, and the results are given in Table 1 below.

**Table 1: PTX loading efficacy in Lipiodol BeadBlock ***

| Sample | Target loading (mg) | Actual loading (mg) | Loading efficacy (%) |
|---|---|---|---|
| 1 (500-700 µm) | 21.4 | 2.2 | 10.2 |
| 2 (500-700 µm) | 38.8 | 6.3 | 16.4 |
| 3 (700-900 µm) | 21.0 | 7.4 | 35.2 |
| 4 (700-900 µm) | 48.8 | 16.7 | 34.2 |

| | | | |
|---|---|---|---|
| *Lipiodol is 23 % (v/v) in loading solution | | | |

### Example 12: Elution of PTX from Lipiodol Bead Block

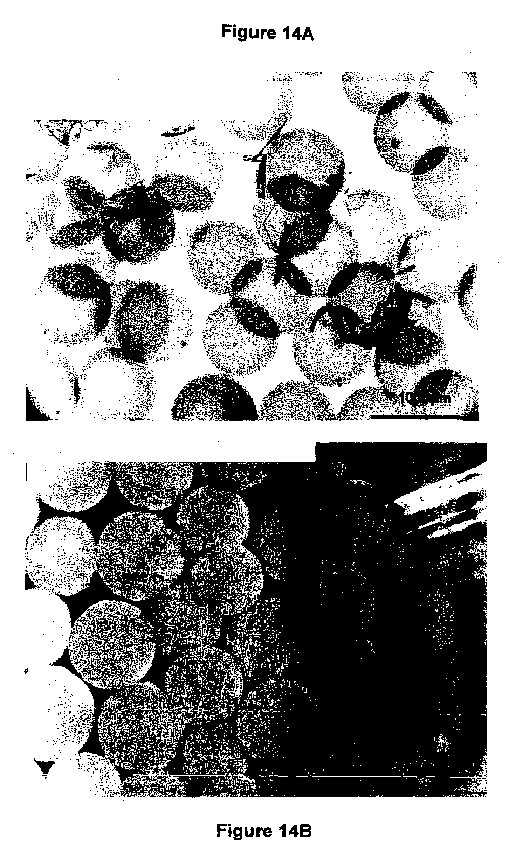

Samples 3 and 4 from Example 11 were used for the PTX elution test. 1.2 ml of each sample was mixed with 200 ml PBS buffer on a roller mixer under room temperature. At predetermined time interval, 50 or 100 ml solution was removed and 50 or 100 ml fresh PBS was added. The elution profile after HPLC processing is shown in Figure13.

### Example 13: Loading and elution of Dexamethasone (Dex) using Lipiodol

Dex loading solution (5mg/ml) in ethanol was prepared. Using this solution Bead Block beads were loaded as described in Example 6. As a control, additional beads were loaded with Dex ethanol solution without the use of Lipiodol.

Images showed that samples loaded without the use of Lipiodol did not uptake Dex. Crystals of Dex were present on the beads surface (Fig. 14A). Beads loaded using IBU-Lipiodol were opaque and no crystals were apparent on beads surface (Fig. 14B).

### Example 14: Loading and elution using different beads

Two types of ion-exchange resins (Amberlyst (SIGMA, sulphonic acid based, 1.9mg/ml exchange capacity) and Amberlite (Sigma, IRA700 (chloride) quaternary ammonium-group containing styrene-DVB resin having total exchange capacity of 1.4mg/ml, density 0.7g/ml)) were loaded with IBU-Lipiodol. The loading and elution procedure was as described in Example 6 using a solution containing 100mg/ml IBU.

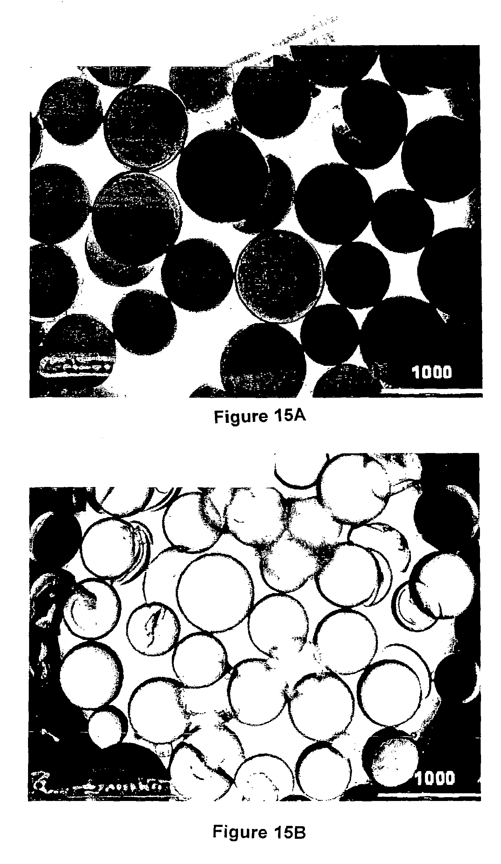

Amberlite uptake was <1mg/ml bead of IBU (Fig. 15A). Amberlyst was loaded with IBU ~15mg/ml (Fig. 15B).

## Claims

1. A process for forming stable radiopaque embolic beads including the steps:
a) contacting substantially dry water-swellable beads, which are capable of swelling in phosphate buffered saline at 20°C to an equilibrium water content of at least 40%, with a loading liquid comprising a pharmaceutically acceptable iodinated oil for a period of at least 5s to produce oil-loaded beads.
b) recovering the oil-loaded beads from any excess extra-particulate loading liquid;
c) contacting the beads after step b) with an aqueous storage liquid in excess of the amount required to swell the beads to equilibrium; and
d) sterilising the beads in the storage liquid by heating to a temperature of at least 90°C for at least 5 seconds.

2. A process according to claim 1 in which the loading liquid also comprises an organic solvent which is miscible or emulsifiable with the oil.

3. A process according to claim 2 in which the organic solvent is capable of swelling the beads and is volatile relative to the oil.

4. A process according to claim 2 or claim 3 in which the organic solvent is selected from C₁₋₁₂ alkanol, preferably ethanol and propanol, more preferably ethanol.

5. A process according to any of claims 2 to 4 in which after step c) the oil-loaded beads are dried to remove substantially all the organic solvent by evaporation.

6. A process according to any preceding claim in which the iodinated oil contains at least 30% by weight iodine, preferably 35 to 45% iodine.

7. A process according to claim 6 in which the iodinated oil is an iodinated mixture of a naturally occurring oil, preferably comprising primarily lower alkyl esters of iodinated stearic acid.

8. A process according to any preceding claim comprising an additional step prior to step a) of subjecting beads swollen with an aqueous liquid to a drying step, preferably including lyophilisation.

9. A process according to any preceding claim in which in step a) the dry beads are contacted with the loading liquid for a period of at least 30s, preferably at least 5 min, more preferably at least 30 min, often at least one hour.

10. A process according to any preceding claim in which the step d) sterilisation is conducted by heating, preferably at a temperature of at least 100°C for a period of at least 5 seconds, more preferably by heating at a temperature of 121°C in an autoclave, for at least 15 minutes.

11. A process according to any preceding claim in which the polymer has alcoholic hydroxyl substituent or acylated derivatives thereof.

12. A process according to claim 11 in which the polymer is crosslinked poly(vinyl)alcohol, preferably formed by copolymerising ethylenically unsaturated poly(vinyl)alcohol macromer with ethylenically unsaturated comonomer.

13. A process according to claim 11 or 12, in which the iodinated oil is an ester, including a step involving trans- or inter-esterification between the hydroxyl group on the polymer and the ester group of the iodinated oil whereby iodinated acyl groups become bound to the polymer through ester linkages.

14. A process according to any preceding claim in which the swellable beads are substantially spherical in shape.

15. A process according to any preceding claim in which at the start of the process the beads have sizes such that, when swollen to equilibrium in phosphate buffered saline at 20°C, the average diameter is in the range 50 to 1500 µm, preferably in the range 100 to 1200 µm.

16. A process according to any preceding claim in which the loading liquid also contains a drug.

17. A hydrophilic polymer having at least one pendant iodinated C₈₋₂₄-alkanoyl group.

18. A polymer according to claim 17 which is in the form of a cross-linked water-swellable, water-insoluble material.

19. A polymer according to claim 18 which is in particulate form, preferably microsphere form.

20. A polymer according to claim 19 in which the particles have average sizes in the range 50 to 2000µm, preferably in the range 100 to 1500µm, more preferably in the range 150 to 1200µm.

21. A polymer according to any of claims 17 to 20 which is a poly(vinyl alcohol) polymer.
